Europäisches Patentamt

European Patent Office

Office européen des brevets

(11) Veröffentlichungsnummer: **0 444 243 A2**

(12) **EUROPÄISCHE PATENTANMELDUNG**

(21) Anmeldenummer: 90120688.8

(22) Anmeldetag: 29.10.90

(51) Int. Cl.⁵: **G01F 1/32**, G01F 1/66, A61B 5/087

(30) Priorität: 30.10.89 DE 3936026

(43) Veröffentlichungstag der Anmeldung: 04.09.91 Patentblatt 91/36

(84) Benannte Vertragsstaaten: AT BE CH DE DK ES FR GB GR IT LI LU NL SE

(71) Anmelder: **Reents, Heinrich, Prof. Dr. Ing.**
**Magnolienweg 23**
**W-4750 Unna(DE)**

(72) Erfinder: **Reents, Heinrich, Prof. Dr. Ing.**
**Magnolienweg 23**
**W-4750 Unna(DE)**

(54) **Verfahren mit den dazugehörigen Vorrichtungen zur Messung und Analyse von Gasströmungen und deren Richtung.**

(57) Die exakte Messung von Atemströmungen ist eine Voraussetzung zur Erkennung und Heilung von Atemstörungen. Die am Markt befindlichen Geräte sind zu ungenau - sie gestatten keine Auswertung nach unterschiedlichen Kriterien.

Gegenstand der Erfindung ist ein Verfahren zur Messung von Gasströmungen und Strömungsrichtungen, das auf einer Schwingungsanalyse beruht. Die Vorrichtungen zur Durchführung des Verfahrens führen zu extrem preiswerten, sehr exakt messenden Sensoren, die leicht austauschbar sind, keine elektrisch leitenden Verbindungen benötigen und im Falle starker Kontamination als Einwegsensoren eingesetzt werden können. Die Sensoren werden kombiniert mit mobilen Auswerteeinheiten, die ausgerüstet mit Microcontrollern und eigener Intelligenz eine Vielzahl von Anwendungsmöglichkeiten erschließen.

EP 0 444 243 A2

Die exakte Messung von Atemströmungen ist eine Voraussetzung zur Erkennung und Heilung von Atemstörungen. Die am Markt befindlichen mobilen Systeme sind relativ ungenau. Sie gestatten die Aufzeichnung nur eines Wertes. Sie besitzen kein Speichervermögen und sind nicht in der Lage,Manipulationen zu erkennen.

Der Arzt benötigt jedoch eine Vielzahl von Meßwerten wie Vitalkapazität, forcierte Vitalkapazität, Atemstoßtest, Atemstoßtestrelativ, Atemgrenzwert, forcierter Atemstrom, forcierter mittlerer Atemstrom, forcierte mittlere Atemstromzeit, Kurvenverlauf des Einatmungsstroms sowie des Ausatmungsstroms.

Ein weiteres Problem stellt die Empfindlichkeit der bekannten Meßvorrichtungen dar. Während sie einsatzfähig sind für Erwachsene, läßt die Genauigkeit der Atemüberwachung von Kleinkindern und Neugeborenen zu wünschen übrig.

Neben der fehlenden Empfindlichkeit reicht die Reproduzierbarkeit den Anforderungen der Ärzte nicht.

Ein weiteres Problem ist die Kontamination. In der Praxis kommt es darauf an, schnell austauschbare Meßfühler zu hab en, die steril sind in Form eines Einwegmeßfühlers oder nach Gebrauch wieder sterilisiert werden können.

Die Aufgabenstellung der Erfindung besteht darin, eine Meßeinheit für Gasströme zu entwickeln, die folgenden Anforderungen gerecht wird:

1. Das System muß einfach in der Bedienung sein.

2. Es muß leicht sein, tragbar und von geringen Abmessungen.

3. Für den mobilen Einsatz muß der Energiebedarf gering sein.

4. Kontaminierte Teile des Gerätes müssen leicht zu reinigen sein z.B. durch leicht austauschbare Strömungssensoren

5. Das System muß in der Lage sein, Strömungen in beiden Richtungen zu messen - Einatmen, Ausatmen

6. Die Strömungsrichtung muß automatisch erkannt werden und der Auswerteeinheit gemeldet werden.

7. Die Empfindlichkeit muß hoch sein über einen weiten Bereich.

8. Verschiedene Anforderungen an die Empfindlichkeit müssen automatisch erkannt werden

9. Daten müssen intern abgespeichert werden können und nach Bedarf abrufbar sein.

10. Der Zeitpunkt der Datenaufnahme muß automatisch erfaßt und abgespeichert werden.

11. Die Meßwerte müssen reproduzierbar sein.

12. Die Auswertung der Daten muß auch in Korrespondenz mit anderen Rechnern möglich sein.

13. Das System muß wirtschaftlich in der Herstellung sein und den erhöhten Anforderungen der Umwelt gerecht werden.

Diesen Anforderungen wird die vorgestellte Erfindung gerecht.

Das Meßprinzip der im Handel befindlichen Geräte basiert auf dem Prinzip des Schleppzeigers. Die Genauigkeit und Reproduzierbarkeit der Meßwerte läßt bei diesem System zu wünschen übrig. Weiterhin sind Geräte bekannt, die die Meßergebnisse mit Hilfe von Radialflügelrädern, die sich gänzlich im Luftstrom befinden, erfassen und auswerten. Sensoren dieser Systeme sind jedoch mit der Auswerteeinheit fest verbunden. Der Nachteile dieser Systeme liegt

a) in der Schwierigkeit , die kontaminierten Teile zu reinigen

b) in der Beeinflussung des Atemstroms durch die Meßvorrichtung

c) im relativ hohen Energieverbrauch

d) der fehlenden Möglichkeit, Daten abzuspeichern, sie nach anderen Kriterien auszuwerten, der Prüfung über Manipulationen.

Das vorgestellte Verfahren mit den dazu gehörigen Vorrichtungen arbeitet wie folgt:

In einem Meßrohr werden ein bzw. zwei Stäbe definierter Geometrie und definierten Materials eingebracht. Durch den Gasstrom/Luftstrom fängt dieser Stab, fangen diese Stäbe an zu schwingen. Das Schwingungsverhalten (Amplitude/Frequenz) ändert sich bei sich änder nden Gasströmungen.

Der Stab hat derart kleine Abmessungen, daß er das Meßergebnis nur unwesentlich beeinflußt. Er hat jedoch sein eigenes Resonanzverhalten. Es unterscheidet sich von dem des Meßrohres wesentlich. Berührungen des Meßrohres haben damit keinen Einfluß auf das Meßergebnis.

Das Signal wird über den Stab aus dem Meßrohr oder an die Meßrohrwandung geleitet. Das Meßrohr wird im Bereich des Stabes mit einer schalldichten/schallgedämmten Kammer verbunden. Von einem Schwingungswandler werden in der Kammer die Meßwerte aufgezeichnet und der Auswerteeinheit zugeführt.

Der Schwingungsaufnehmer kann ausgeführt sein in Form eines Piezofühlers, eines kapazitiven Schallwandlers (Kondensatormikrophon) oder eines induktiven/elektrodynamischen Schallwandlers. Eine schalldichte/schallgedämmte Kammer kann preiswert erzeugt werden durch eine flexible Schaumstoffauflage, die gelöchert ist und an die der Sensor angelegt wird.

Durch die Anwendung dieses Verfahrens können die Strömungssensoren leicht von der Aufnahme- und Auswerteeinheit entkoppelt werden. Sie benötigen nur eine Vorrichtung zur Befestigung des Sensors und zum lagerichtigen Einlegen des Sensors. Beispiele dieser Vorrichtungen sind einfache Klammern, ein Klappmechanismus oder Nasen, die am Meßrohr angebracht sind.

Der einfache Aufbau des Meßrohres gestattet eine leichte Reinigung von Kontaminationen, im Extremfall durch die Verwendung eines Einwegsensors entsprechend einer Einwegspritze.

Das Meßsignal ist leicht digitalisierbar. Es kann durch die Verwendung extrem steiler Filter gegen äußere Umwelteinflüsse/Umgebungseinflüsse leicht geschützt werden. Die Empfindlichkeit kann durch eine automatische Verstärkungsregelung unterschiedlichen Anwendungsfällen angepaßt werden.

Eine einfache Ausführungsform der automatischen Verstärkungsregelung läßt sich realisieren durch eine mechanische, optische, induktive oder elektronische Codierung des Meßrohrs. Kinder haben eine kleinere Mundöffnung und erreichen mit ihrem Atemstrom geringere Strömungsgeschwindigkeiten als Erwachsene. Durch Austausch des Meßrohres mit Codierung erfolgt in der Auswerteeinheit eine automatische Umstellung. Diese Umstellung kann auch sinnvoll sein bei der Anwendung des Meßsystems bei verschiedenen Gasen oder Flüssigkeiten. In einer weitere Variante kann die automatische Verstärkungsregelung durch den in der Auswerteeinheit vorhandenen Mikrocontroller selbsttätig veranlaßt werden durch eine Analyse des Eingangssignals.

Ein entscheidender Vorteil des oben beschriebenen Verfahrens liegt in der Verschleißfestigkeit. Es gibt keine sich mechanisch drehenden Teile.

Ein Gasstrom setzt sich richtungsgebunden fort. Kommt im Meßrohr nun ein zweiter Stab zum Einsatz, wird beim Ein- oder Ausatmen einer der beiden Stäbe zuerst in Schwingungen versetzt. Durch dieses Verfahren mit zwei Stäben ist eine automatische Richtungserkennung möglich.

Diese Richtungserkennung ist z.B. notwendig beim Messen der sogenannten Vitalkapazität. Diese ist definiert als das Lungenvolumen nach maximaler Inspiration und maximaler Expiration- also der Summe des Einatmungsvolumens und des Ausatmungsvolumens.

Der Vorteil des Verfahrens liegt neben der in starkem Maße steigerbaren Empfindlichkeit in der Genauigkeit und Miniaturisierungsmöglichkeit. Diese Voraussetzungen sind zu erfüllen, will man das System handhabbar für den Benutzer konzipieren. Durch die Digitalisierung der Meßwerte ist eine direkte Ankopplung des Sensors an Microcontroller möglich. Beliebige Meßkurven können erfaßt und ausgewertet werden nach jedem vorgegebenen funktionalen Zusammenhang.

Meßwerte können nahezu stetig gespeichert werden, sie können angezeigt und nach Bedarf an korrespondierende Rechner weitergeleitet werden.

Dies ist zum Beispiel wichtig bei der graphischen Darstellung des Ein- und Ausatmungsstroms. Für diesen Zweck ist in jeder Einheit eine Schnittstelle (seriell oder parallel) zu einem übergeordneten Rechner vorgesehen.

Der REchner gestattet die Prüfung der Werte - er kann z.B. feststellen, inwieweit Ein- und Ausatmungsströme bewußt verfälscht wurden. Durch eine Kombination mit einer Echtzeituhr kann darüberhinaus der Inspirations- und Expirationsvorgang mit der Tageszeit korreliert werden.

In einer anderen Ausführungsvariante wird der Gasstrom durch ein Flügelrad gemessen, das in das Meßrohr hineinragt. Das Flügelrad ist ein Bestandteil des Sensors - dadurch gestattet auch diese Ausführungsform eine kompakte Sensoreinheit- im Extremfall bis zum Einwegsensor.

Die Umdrehung des Rades sind ein Maß für die Strömungsgeschwindigkeit. Die Abtastung erfolgt in dieser Variante optoelektronisch.

Durch eine versetzte Anbringung der Durchbohrungen des Flügelrades auf verschiedenen Radien in verschiedenen Proportionen kann auch die Strömungsrichtung gleichzeitig erkannt werden; in einer anderen Variante ist die Länge der Durchbohrungen unterschiedlich auf einem unterschiedlichen Radius. Der Rechner kann erkennen, ob erst ein kurzer Impuls gelesen wird und dann ein langer Impulsdies kann z.B. der Linksdrehung entsprechen. Erfolgt die Impulsfolge umgekehrt, kann dies ein Maß sein für Richtungsänderungen.

Durch die mechanische Reibung des Flügelrades mit der Welle können jedoch bei dieser Lösung bei hohen Drehzahlen Ungenauigkeiten auftreten (Reibungskoeffizient, Rattern). Der Sensor ist in dieser Ausführungsform nur bedingt verschleißfest. Die Herstellkosten werden mit Sicherheit höher liegen als die der ersten Variante (Meßrohr mit Stab).

Der Gegenstand der Erfindung kann überall angewendet werden, wo bestimmte Strömungen von Gasen auch in Extremwerten gemessen werden sollen. Geeignete Einsatzgebiete sind dementsprechend neben der Medizintechnik der Bereich der Gastechnik, der Messung der Windstärke und -richtung, der Alarmerfassung beim unbefugten Betreten von Räumen, der Prozeßtechnik, der Kraftfahrzeugsensorik. Darüberhinaus ist das System auch geeignet zur Strömungsmessung von Flüssigkeiten z.B. bei Heizkreisläufen.

Ausführungsformen der Erfindung sollen im folgenden durch die beigefügten Bilder erläutert werden:

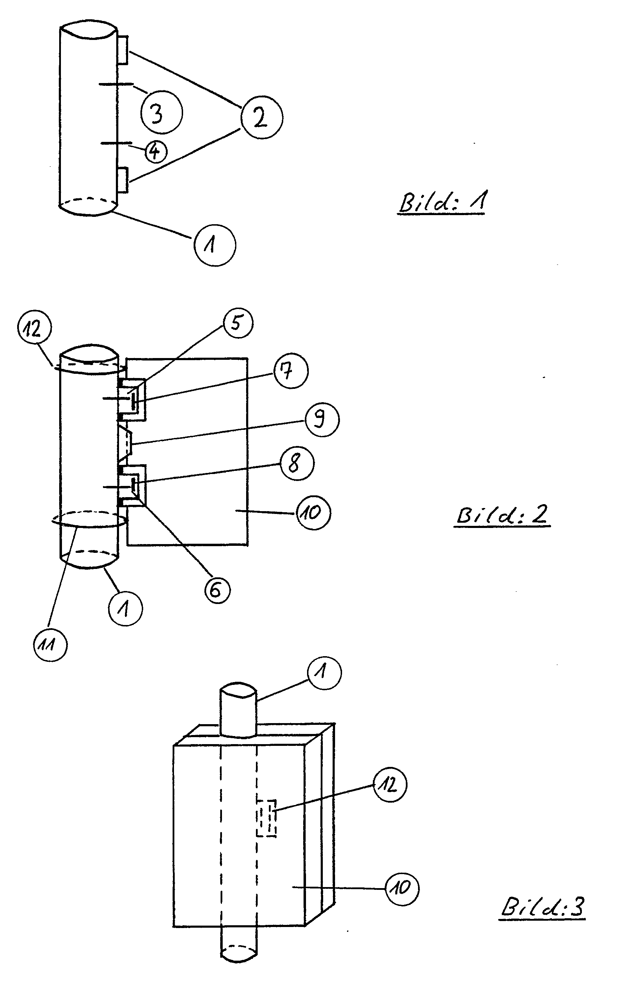

Bild 1: zeigt das Meßrohr (1). Zur Lagefixierung sind am Meßrohr Fixiereinrichtungen (2) angebracht. (3) kennzeichnet den Stab definierter Geometrie, der durch den Luftstrom in Schwingungen versetzt wird. Er kann aus verschiedenen Materialen bestehen. Dieser Sensortyp entspricht dem Einwegsensor. Nach Gebrauch kann er aus Gründen der Kontamination aus dem Gerät entfernt werden. Diese Ausführungsform ist in der Lage, Strömungsgeschwindigkeiten in jeder Richtung zu messen.

Kombiniert man diesen Sensor mit einem zweiten Stab (4), ist die Strömungsrichtung feststellbar. Dies ist dadurch bedingt, daß sich eine Druckwelle fortsetzt. Durch extrem schnelle Schaltglieder kann festgestellt werden, welcher der Sensoren zum ersten Mal von der Druckwelle erfaßt wurde.

Wird in dieser Ausführungsform der erste Stab strömungsrichtungsabhängig unterschiedlich geformt, kann auf den zweiten Stab auch in einer anderen Ausführungsvariante verzichtet werden. Diese Variante wird erst realisierbar sein, wenn die Rechengeschwindigkeit der Microcontroller erheblich gesteigert wird bei annehmbaren Kosten.

Bild 2 zeigt den Sensor (1) angekoppelt an die Auswerteeinheit. Man erkennt die schalldichte/schallgedämmte Kammer (5 und 6) und den Schwingungswandler (7 und 8). Die Fixiervorrichtungen bestehen in dieser Ausführungsvariante aus einer Nase (9), die ihr Gegenstück im Geäuse (10) findet sowie Halteklammern (11 und 12). Die Halteklammern umschließen den Sensor in dieser Variante nur zum Teil.

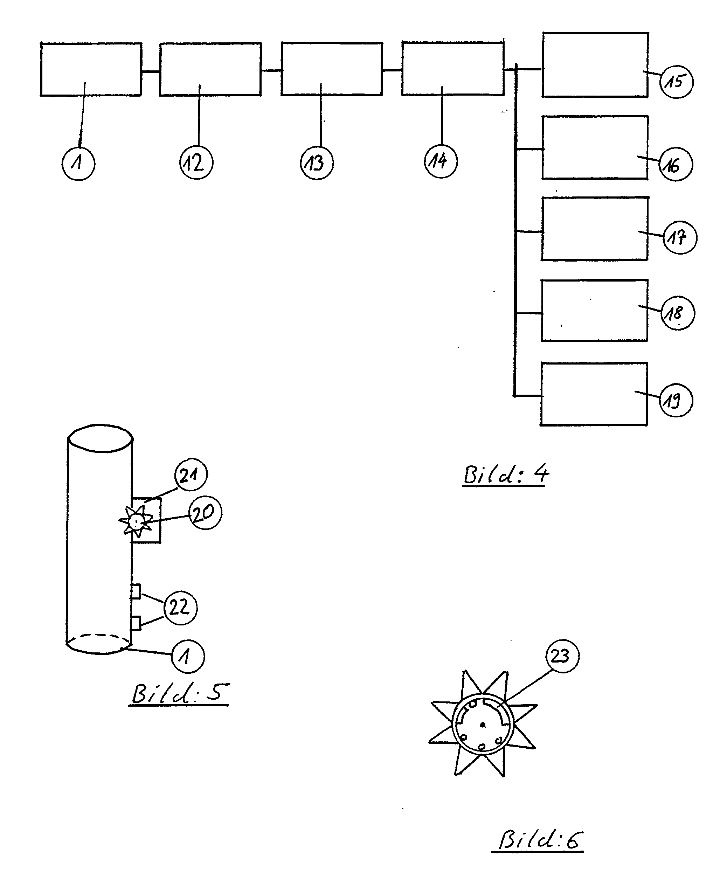

In einer weiteren Variante (Bild 3) wird der Sensor mittig im Gehäuse fixiert. Das Gehäuse wird einfach aufgeklappt, oder der Sensor wird in das Gehäuse geschoben, oder alternativ von hinten am Gehäuse befestigt.

Diese Variante hat den Vorteil, daß sie sowohl von einem Links-, als auch von einem Rechtshänder gut zu handhaben ist. Im Gehäuse ist der Schailwandler untergebracht(12), der Verstärker mit seinen Filtern und Digitalisierungsgliedern, Microcontroller etc.

Bild 4 zeigt die Komponenten des mobilen Meßgerätes. Die Kopplung des Sensors (1) mit der Meßeinheit erfolgt über die schalldichte/ schallgedämmte Kammer. Die nach außen geführten Schwingungen werden über den Schallwandler oder in der alternativen Ausführungsform optoelektronischen Wandler (12) erfaßt, durch Verstärker, Filter, Schmitt-Trigger aufbereitet(13) und dem Microcontroller (14) zugeführt. Je nach Anwendungsfall wird dieser Microcontroller, der ja ein eigenständiger REchner ist, ergänzt um externe Speicher (15) - zum Aufnehmen der Meßdaten/Patientendaten über einen längeren Zeitraum -, um Anzeigen (16), Schnittstellen zu übergeordneten Rechnern (17), Echtzeituhr (18) und Funktionstasten (19).

Über die Schnittstellen können Daten abgerufen werden von der mobilen Station zu übergeordneten Rechnern. Dort können Plausibilitätsüberprüfungen durchgeführt werden, die Kurvenverläufe der Ein-und Ausatmungsströme graphisch dargestellt werden, die Meßwerte berechnet und die Ergebnisse ausgedruckt werden.

Bild 5 zeigt einen alternativ verwendbaren Sensor. Man erkennt das Meßrohr (1), das Flügelrad (20). Das Flügelrad läuft in einer durchsichtigen Kammer (21), die durch einen Lichtstrahl von außen abgetastet werden kann. Die Codierung zur Weitergabe der Informationen an die Auswerteeinheit über die gewünschte Empfindlichkeit, das zu messende Gas etc. ist durch (22) dargestellt. Diese Bauweise ermöglicht auch eine Ausführungsform als Einwegsensor.

Die Ankopplung an die Auswerteeinheit erfolgt in dieser Variante über eine optoelektronische Abtastung. Eine scharfe Bündelung des Lichtstrahls ist durch Laserdioden preiswert möglich. Eine Variante der Codierung des Flügelrades zur Erkennung der Strömungsrichtung ist in Bild 6 dargestellt. Durch die unterschiedliche Länge der Durchbohrungen ist dem Rechner es sehr schnell möglich, die Drehrichtung zu erkennen (23). Diese Variante ermöglicht eine Bauform mit nur einer Abtasteinheit.

Die Erfindung ist nicht auf die Ausführungsbeispiele beschränkt, sondern kann im Rahmen der Offenbarung variiert werden. Alle neuen in der Zeichnung und Beschreibung offenbarten Einzel- und Kombinationsmerkmale können als erfindungswesentlich angesehen werden.

**Patentansprüche**

1. Verfahren zur Messung von Gasströmungen dadurch gekennzeichnet, daß ein Gas durch eine mobile Einheit, die keine elektrische Verbindung zu einer Auswerteeinheit hat, geführt wird, das Gas/Luft einen in der Durchströmung befindlichen Stab definierten Materials und definierter Geometrie in Schwingungen versetzt, und der Stab die Schwingung nach Amplitude und Frequenz an die Aussenwandfläche der Meßeinrichtung überträgt, im Übertragungsbereich durch eine schalldichte/schallgedämmte Kammer diese Schwingungen durch einen Schwingungsaufnehmer aufgenommen und einer Auswerteeinheit zugeführt werden.

2. Verfahren nach Anspruch 1 dadurch gekennzeichnet, daß in der mobilen Einheit sich ein zweiter Stab definierten Materials und definierter Geometrie befindet, durch den mittels eines Vergleichs des Schwingungsverhaltens beider Stäbe sich die Strömungsrichtung eindeutig feststellen läßt.

3. Verfahren nach Anspruch 1 und 2 dadurch gekennzeichnet, daß durch die Verwendung angepaßter Filter und Meßverstärker im Rahmen der Signalauswertung äußere Störeinflüsse minimiert werden und alternative Empfindlichkeiten je nach Anwendungsfall eingestellt werden können, im Extremfall bis zu einer automatischen ggfs. rechnergesteuerten Verstärkungsregelung.

4. Verfahren zur Messung von Gasströmungen dadurch gekennzeichnet, daß alternativ zu den Ansprüchen 1 und 2, ein drehbares Flügelrad zum Teil in die Strömung eingeführt wird, dieses durch den Gasstrom in Drehbewegungen versetzt wird, das Flügelrad mit Codierungen versehen wird, die eine automatische Erkennung der Strömungsrichtung ermöglicht und sowohl Drehzahl als auch Drehrichtung optoelektronisch abgetastet werden.

5. Vorrichtung zur Durchführung des Verfahrens nach Anspruch 1-4 dadurch gekennzeichnet, daß ein Meßrohr mit einem oder zwei Stäben definierten Materials und definierter Geometrie versehen wird, die schwingungstechnisch vom Rohr weitgehend entkoppelt sind, in das Meßrohr hineinragen, durch den Gasstrom in Schwingungen versetzt werden, die Schwingungen in eine bzw. zwei schalldichte/ schallgedämmte Kammern berührungslos übertragen werden und dort einer Aufnahme-und Auswerteeinheit zugeführt werden.

6. Vorrichtung zur Durchführung des Verfahrens nach Anspruch 1-4 dadurch gekennzeichnet, daß alternativ zur Vorrichtung 5 ein bewegliches Rad in den Gasstrom zum Teil eingeführt wird, das Rad in sich mit Codierungen versehen ist, die eine Drehrichtung eindeutig erkennen lassen, das Flügelrad durch den Luftstrom in Drehbewegungen versetzt wird und optoelektronisch abgetastet wird.

7. Vorrichtung nach Anspruch 5,6 dadurch gekennzeichnet, daß durch Fixiereinrichtungen die Strömungssensoren eine eindeutige Lage und Befestigung erhalten, sie vom Meß- und Auswertegerät abgenommen werden können, sie damit leicht austauschbar und leicht zu reinigen sind sowie bei Gefahr starker Kontamination als Einwegsensoren zum Einsatz kommen können.

8. Vorrichtung nach Anspruch 5-7 dadurch gekennzeichnet, daß die Sensoren mit mechanischen oder optischen oder induktiven oder elektronischen Codierungen versehen sind, die automatisch Funktionsaufrufe in der Auswerteeinheit auslösen.

9. Vorrichtung nach Anspruch 5-8 dadurch gekennzeichnet, daß die Sensoren mit Meß-, Auswerte-, Anzeige-, Speicher- und Druckeinheiten sowie Datenübertragungseinrichtungen gekoppelt werden können, die eine akustische oder optoelektronische Abtastung einwandfrei ermöglichen.

10. Vorrichtung nach Anspruch 5-9 dadurch gekennzeichnet, daß die Meßeinrichtungen mobil und tragbar sind und mit weiteren Datenverarbeitungseinheiten im Host- und Slave-Betrieb arbeiten können.

Bild: 1

Bild: 2

Bild: 3

Bild: 4

Bild: 5

Bild: 6